# EUROPÄISCHE PATENTANMELDUNG

(11) **EP 2 586 363 A1**
(43) Veröffentlichungstag der Anmeldung: **01.05.2013**
(21) Anmeldenummer: 12188614.7
(22) Anmeldetag: 16.10.2012
(51) Int. Cl.: A61B 5/00

(54) **Katheter und Katheteranordnung**

(30) Priorität: 24.10.2011 US 201161550470 P
(71) Anmelder: VascoMed GmbH, 79589 Binzen (DE)
(72) Erfinder: Fandrey, Stephan, 8910 Affoltern am Albis (CH); Weiss, Ingo, 12435 Berlin (DE); Knorr, Stefan, 10119 Berlin (DE)
(74) Vertreter: Lindner-Vogt, Karin L.

(57) **Zusammenfassung**

Katheter (1) mit einem langgestreckten und axial starren Katheterkörper (5), der in Bezug auf eine Gebrauchslage ein distales und ein proximales Ende hat, wobei am distalen Ende ein mit einem vorbestimmten Bewegungs- oder Verformungswiderstand versehenes bewegliches und/oder verformbares Schutzelement (7) oder ein entsprechender Schutz-Abschnitt des Katheterkörpers angeordnet ist und ortsfest am Schutzelement oder Schutz-Abschnitt Gebermittel (9) einer Wandlereinrichtung zur Wandlung einer Lageverschiebung in eine nichtmechanische Größe und am Katheterkörper korrespondierende Aufnehmermittel (11) dieser Wandlereinrichtung, oder umgekehrt, vorgesehen sind und der Katheterkörper eine Leitung (13, 15) zur Fortleitung eines von der Wandlereinrichtung erzeugten Messsignals zum proximalen Ende sowie einen Messanschluss am proximalen Ende aufweist.

## Beschreibung

Die Erfindung betrifft einen Katheter mit einem langgestreckten Katheterkörper, der in Bezug auf eine Gebrauchslage ein distales und ein proximales Ende hat. Sie betrifft des Weiteren eine Katheteranordnung mit einem solchen Katheter und einer daran angeschlossenen Messeinrichtung.

In der medizinischen Praxis ist eine Vielzahl verschieden konstruierter Katheter oder katheterähnlicher Einrichtungen (z.B. Elektrodenleitungen) bekannt und in massenhaftem Gebrauch. Sie werden teilweise von erfahrenen Spezialisten, teilweise aber auch von Ärzten oder auch medizinischem Personal ohne spezielle Kenntnisse und Erfahrung angewendet. Schädigungen oder Beeinträchtigungen des Patienten müssen gleichwohl zuverlässig ausgeschlossen werden.

Beim Einsatz bekannter Katheter mit einer Kunststoff- oder Metallspitze tritt eine Perforationsgefahr auf. Um die Flächenpressung und damit die Perforationsgefahr gering zu halten, müssen bei der Steifheit des Katheterschaftes und der Katheterspitze Kompromisse eingegangen werden. Diese Kompromisse schränken u. U. die Manövrierfähigkeit und die Lagestabilität des Katheters ein. Zudem sind herkömmliche Katheter oder Elektrodenleitungen "blind" bei Wandungskontakt in einem Gefäß oder Hohlorgan, d. h. sie können dem Bediener keine den Kontakt betreffenden Informationen liefern. Er ist dabei bei der Bewertung der Situation auf sein manuelles Geschick und - soweit verfügbar - Röntgenkontrolle angewiesen.

Seit einigen Jahren werden Entwicklungen betrieben, die auf die Realisierung einer Berührungs- bzw. Kraftsensorfunktion an einer distalen Katheterspitze abzielen. Aus der WO 2005/01511 A1 ist ein entsprechender Kraftsensor auf der Basis von Lichtwellenleitern bekannt, bei dem über einen ersten Lichtleiter zur Katheterspitze übertragenes und über einen zweiten Lichtleiter von der Spitze zurückgeleitetes Licht in einem Verformungsbereich an der Katheterspitze durch Reflexion moduliert und hierdurch eine Berührung detektiert wird. Aus der WO 2008/003307 A2 ist ein elektrisch wirkender Kraftsensor zum Erfassen eines Kraftvektors bekannt, der ebenfalls an einem Katheter oder einer ähnlichen medizintechnischen Einrichtung eingesetzt werden kann.

Auf eine andere Entwicklergruppe geht eine Reihe von Druckschriften zurück, in denen ein dreiachsiger faseroptischer Kraftsensor bzw. ein mit einem solchen ausgestatteter Katheter beschrieben wird; vgl. etwa US 2008/0009750 A1, WO 2009/007857 A2 oder US 2009/0177095 A1. Die in diesem Kontext eingesetzte Lichtleiter-Kraftsensorik arbeitet auf der Grundlage des Fabry-Perot-Prinzips mit einem in Längsrichtung segmentierten Zylinder im distalen Endbereich des Katheterkörpers und nutzt zum Nachweis von Berührungen an der Katheterspitze Änderungen des Reflexionsverhaltens an den Spalten zwischen den Zylindersegmenten.

Der Erfindung liegt die Aufgabe zugrunde, einen verbesserten Katheter bereitzustellen, der ohne die Gefahr von Schädigungen oder Beeinträchtigungen des Patienten auch von weniger Geübten eingesetzt werden kann, dessen Konstruktion dennoch ohne weiteres den im klinischen Einsatz bestehenden Anforderungen gerecht wird und dessen Gebrauchswert insgesamt gesteigert ist.

Diese Aufgabe wird durch einen Katheter mit den Merkmalen des Anspruchs 1 gelöst. Mit der Erfindung wird des Weiteren eine Katheteranordnung mit den Merkmalen des Anspruchs 13 bereitgestellt.

Die Erfindung geht von dem Gedanken aus, bekannte Katheteraufbauten durch ein spezielles bewegliches und/oder verformbares Schutzelement (bzw. einen entsprechend ausgebildeten Schutz-Abschnitt des Katheterkörpers) am distalen Ende des Katheters zu modifizieren. Dieses Schutzelement oder dieser Schutz-Abschnitt ist, gemäß einer weiteren Überlegung der Erfinder, mit einem vorbestimmten Bewegungs- bzw. Verformungswiderstand zu versehen. Weiterhin gehört zur Erfindung der Gedanke, aus der Bewegung bzw. Verformung des Schutzelements oder Schutz-Abschnitts über geeignete Messmittel Daten zu gewinnen, die eine verbesserte Beurteilung der konkreten Einsatzsituation ermöglichen. Hierzu ist eine Geber-/Aufnehmer-Konfiguration bezüglich des axial starren Katheterkörpers einerseits und bezüglich des beweglichen oder verformbaren Schutzelements oder Schutz-Abschnitts derart verteilt zu positionieren, dass sie relevante Bewegungen oder Verformungen hinreichend erfasst.

In einer Ausführung der Erfindung ist der vorbestimmte Bewegungs- oder Verformungswiderstand durch eine Federkraft einer elastischen Anbringung des Schutzelements oder eine Eigen-Elastizität des Schutzabschnitts realisiert. Es kann also ein spezielles Halterungs- bzw. Aufhängungselement mit geeignet gewählter Feder- bzw. Elastizitätskonstante vorgesehen sein, oder es ist ein verformbares Schutzelement oder ein verformbarer Schutz-Abschnitt des distalen Katheterendes durch geeignete Materialwahl und Dimensionierung mit der gewünschten Eigen-Elastizität versehen.

In weiteren bevorzugten Ausführungen sind mindestens drei Geber- oder Aufnehmerelemente um eine Längsachse oder virtuell verlängerte Längsachse des Katheterkörpers verteilt, insbesondere gleichmäßig verteilt, um eine Lageverschiebung des Schutzelements oder Schutz-Abschnitts vektoriell zu erfassen. In einer ersten Ausgestaltung dieses Prinzips sind genau einem Geberelement genau drei Aufnehmerelemente, die insbesondere gleichmäßig mit einem Winkelabstand von je 120° um die Längsachse verteilt sind, oder ortsaufgelöst empfangende Aufnehmeranordnungen zugeordnet. Eine andere Ausgestaltung sieht vor, dass genau drei Geberelemente, die insbesondere gleichmäßig mit einem Winkelabstand von je 120° um die Längsachse verteilt sind, einer ortsaufgelöst empfangenden Aufnehmeranordnung zugeordnet sind.

In weiteren Ausführungen der Erfindung weisen die Gebermittel ein lichtemittierendes Element und die Aufnehmermittel, insbesondere auch jedes Aufnehmerelement in einer Aufnehmeranordnung, ein Fotodetektorelement auf. Hierbei kann insbesondere die Aufnehmeranordnung eine CCD-Zeile oder -Matrix aufweisen. In weiteren Ausgestaltungen ist vorgesehen, dass dem oder mindestens einem lichtemittierenden Element oder dem oder mindestens einem Fotodetektor ein flächiger Farbfilter oder Polarisationsfilter mit lokal variierender Filtercharakteristik zugeordnet ist. Darüber hinaus sind weitere Ausgestaltungen des optischen Erfassungsprinzips möglich, auf die weiter unten Hinweise gegeben werden.

In einer anderen grundsätzlichen Ausführung weisen die Gebermittel und die Aufnehmermittel jeweils mindestens eine Elektrode oder einen elektrischen Kontakt zur Einprägung einer Messspannung und zur Erfassung eines Messstromes oder umgekehrt auf. In zweckmäßigen Ausgestaltungen hat ein solcher Katheter mindestens ein lineares oder flächiges Elektroden- oder Widerstands-Array und einen im wesentlichen punktförmigen Abtast-Kontakt oder kapazitiven Abgriff als Geber- und Aufnehmermittel, wobei einer von Elektroden- bzw. Widerstands-Array oder Abtast-Kontakt ortsfest am starren Katheterkörper und das andere ortsfest am Schutzelement oder Schutz-Abschnitt angebracht ist. Auch im Rahmen dieses Messprinzips sind weitere Ausgestaltungen möglich, auf die weiter unten hingewiesen wird.

In weiteren Implementierungen sind den Geber- oder Aufnehmermitteln Zeitsteuermittel zu einer vorbestimmten zeitlichen Steuerung ihrer Aktivierung oder ihres Ortes auf einer vorbestimmten Bewegungsbahn im Bereich des Schutzelements oder Schutz-Abschnitts zugeordnet und die Aufnehmermittel zur zeitaufgelösten Signalaufnahme ausgebildet.

In einer Realisierungsform von großer praktischer Bedeutung ist der Katheter ausgebildet als Elektrodenleitung mit mindestens einer Stimulations- und/oder Abfühlelektrode. Erfindungsgemäße Katheter können aber auch als Ablationskatheter zum Anschluss an eine Hochfrequenzeinrichtung oder als rein mechanisch wirkende Katheter, etwa als Dilatations- oder Stent-Einführkatheter, ausgeführt sein.

Eine erfindungsgemäße Katheteranordnung umfasst neben einem erfindungsgemäßen Katheter eine an den Messanschluss angeschlossenen Messeinrichtung zur Bestimmung einer Lageverschiebung des Schutzelements oder Schutz-Abschnittes und/oder einer hierauf einwirkenden Andruckkraft. Zweckmäßige Ausgestaltungen der erwähnten Messeinrichtung ergeben sich weitgehend aus den oben genannten unterschiedlichen Ausführungen der eingesetzten Geber-/Aufnehmermittel. In einer speziellen Ausführung sind die weiter oben erwähnten Zeitsteuermittel zu einer vorbestimmten zeitlichen Steuerung der Aktivierung oder des Ortes der Geber- oder Aufnehmermittel als Komponente der an den Katheter angeschlossen Messeinrichtung ausgeführt.

Als Aspekte weiterer Ausführungsformen der Erfindung werden die folgenden genannt; hierbei sind die Gebermittel auch als "Quelle" und die Aufnehmermittel als "Sensor" sowie die Wandlereinrichtung als "Umkodiereinheit" und der Schutz-Abschnitt des Katheters auch als "Mantel" bezeichnet.
- Der Deformationskörper besteht aus zwei zueinander relativ verschiebbaren Teilen (z.B. ein Innenzylinder zum Mantel eines Katheters).
- Es gibt mindestens eine Umkodiereinheit, bevorzugt drei mit 120° Abstand um den Zylinderumfang verteilt.
- Die drei Umkodiereinheiten nutzen denselben Sensor (z.B. im Innenzylinder) und drei Quellen im Mantel.
- Die Umkodierung der (drei) Längsverformungen(en) (z.B. in Folge der Stauchung oder Biegung des Katheters) erfolgt in eine Ortsinformation auf einem Sensor mit Ortsauflösung (z.B. CCD Zeile, CCD Chip, Photodioden(array)).
- Die primäre Ortsinformation (Deformation) wird über einen Filter in eine Frequenzinformation (z.B. Quelle = Licht, Filter = Farbkeil, Sensor = Farbsensor) gewandelt.
- Die Umkodiereinheit kann auch die Natur des Signals zwecks Übertragung wandeln (z.B. Licht in Farbe pro Ort (RGB); über lokalen Sensor; übertragen wird de facto ein elektrisches Signal).
- Die Umkodierung der Ortsinformation erfolgt in eine Phaseninformation.
- Die Umkodierung der Ortsinformation erfolgt in eine Polarisationsinformation (örtlich veränderlicher Polarisationsfilter).
- Die Umkodierung der Ortsinformation erfolgt in eine Zeitinformation (Multiplexverfahren, Scanverfahren; z.B. die Lichtquelle trifft die Position der Sensoren).
- Die Umkodierung erfolgt durch Schleifkontakt, der Sensor ist ein Linearpotentiometer.
- Die Scannung erfolgt durch aktive Linearbewegung der Quelle oder des Sensors.
- Die Scannung erfolgt durch Rotation (z.B. helixförmiger Lichtschlitz auf dem Innenzylinder).
- Die Scannung (Abtastung) erfolgt durch Schleifkontakt und trifft auf einen Sense-Kontakt.
- Zur Detektion der Deformation wird die Kopplung von Spulen genutzt.
- Zur Detektion der Deformation wird eine kapazitive Kopplung genutzt.
- Zur Detektion der Deformation wird eine akustische Kopplung genutzt.
- Zur Detektion der Deformation wird eine resonante Kopplung genutzt.
- Die lineare Position wird mit einer Kratznadel (wie AFM Prinzip) auf einem Kodiermuster gefahren.

Die Erfindung ermöglicht es, jedenfalls in einer oder mehreren Ausführungsformen, einen bestehenden Wandkontakt des Katheters mit der Wand eines Gefäßes oder Hohlorgans messtechnisch zu charakterisieren. So kann die Kraft gemessen werden, mit der der Katheter in einer bestimmten Einführsituation auf das Körpergewebe drückt. In Verbindung hiermit ergeben sich Verbesserungsmöglichkeiten bei Abfühl- wie auch Stimulationsabläufen oder auch bei Ablationsvorgängen, und insbesondere können Perforationsgefahren weitgehend minimiert werden.

Vorteile und Zweckmäßigkeiten der Erfindung ergeben sich im Übrigen aus der nachfolgenden skizzenartigen Beschreibung von Ausführungsbeispielen anhand der Figuren. Von diesen zeigen:
- Fig. 1A und 1B: zeigen jeweils eine Prinzipskizze zur Ausführung der Erfindung,
- Fig. 2: eine Prinzipskizze einer weiteren Ausführungsform,
- Fig. 3: eine Prinzipskizze einer weiteren Ausführungsform,
- Fig. 4: eine Prinzipskizze einer weiteren Ausführungsform,
- Fig. 5: eine Prinzipskizze einer weiteren Ausführungsform und
- Fig. 6: eine Prinzipskizze einer weiteren Ausführungsform.

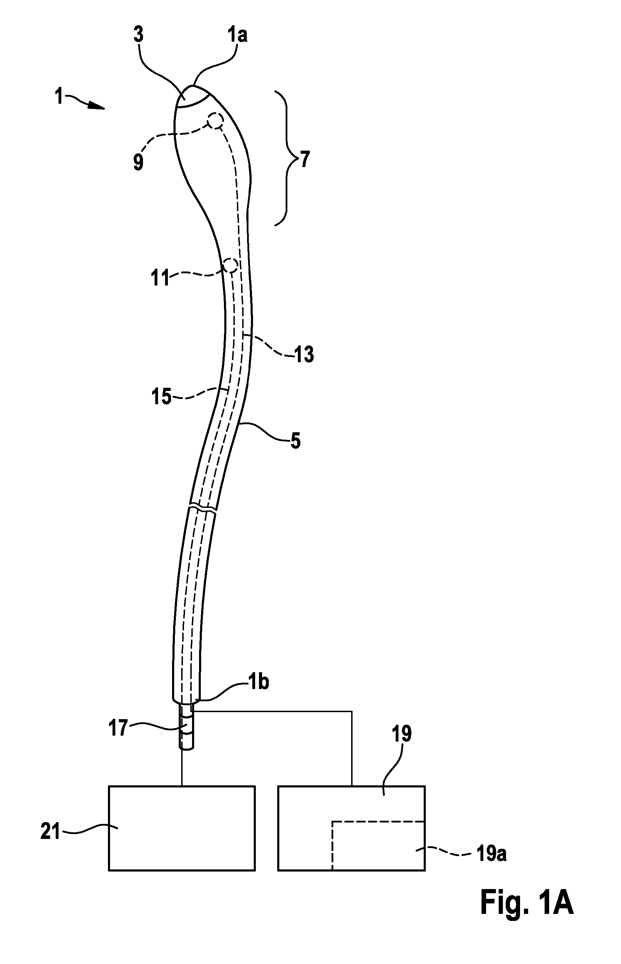

Fig. 1A zeigt schematisch eine Elektrodenleitung 1 als Beispiel eines erfindungsgemäß ausgestalteten Katheters, welche ein distales Ende 1a und ein proximales Ende 1B und am distalen Ende eine Spitzenelektrode 3 aufweist. Die Elektrodenleitung 1 hat einen in axialer Richtung steifen, aber biegsamen Katheterkörper 5, dessen distaler Bereich 7 als verformbarer Schutz-Abschnitt aus einem elastisch deformierbaren Material ausgestaltet ist.

Nahe dem distalen Ende des Schutz-Abschnitts 7 ist ein Geberelement 9 einer Wandlereinrichtung platziert, und am proximalen Ende des Schutz-Abschnitts 7 und somit im distalen Endbereich des starren Abschnitts des Katheterkörpers 5 ist ein angepasstes Aufnehmerelement 11 der Wandlereinrichtung positioniert. Das Geberelement 9 ist über eine Ansteuerleitung 13 und das Aufnehmerelement 11 über eine Messleitung 15, die beide längs durch den Katheterkörper 5 verlaufen, mit einem Anschlussstecker 17 am proximalen Ende verbunden. Über entsprechende Ringkontakte am Anschlussstecker 17 sind die Ansteuerleitung 13 mit einer Mess-Ansteuereinheit 19 und die Messleitung 15 mit einer Messeinrichtung 21 verbunden. In der dargestellten Ausführung umfasst die Mess-Ansteuereinheit 19 eine Zeitsteuerung 19a zur Ausführung eines einen vorbestimmten zeitlichen Ablauf aufweisenden Messprogramms.

Bei der in Fig. 1A dargestellten Katheteranordnung wird eine durch Wandkontakt mit gewisser Andruckkraft hervorgehobene Verformung (Stauchung und/oder Verbiegung) des Schutz-Abschnitts 7 in ein Messsignal des Aufnehmerelements 11 "umkodiert", das Signal wird über die Messleitung 15 zur Messeinrichtung 21 fortgeleitet, und diese gewinnt durch entsprechende Auswertung ein den Wandkontakt der Elektrodenleitung 1 charakterisierendes Auswertungsergebnis, das ein Operateur bei seinem weiteren Vorgehen - etwa für die optimale Festlegung von Platzierung und Andruckkraft der Spitzenelektrode 3 - berücksichtigen kann.

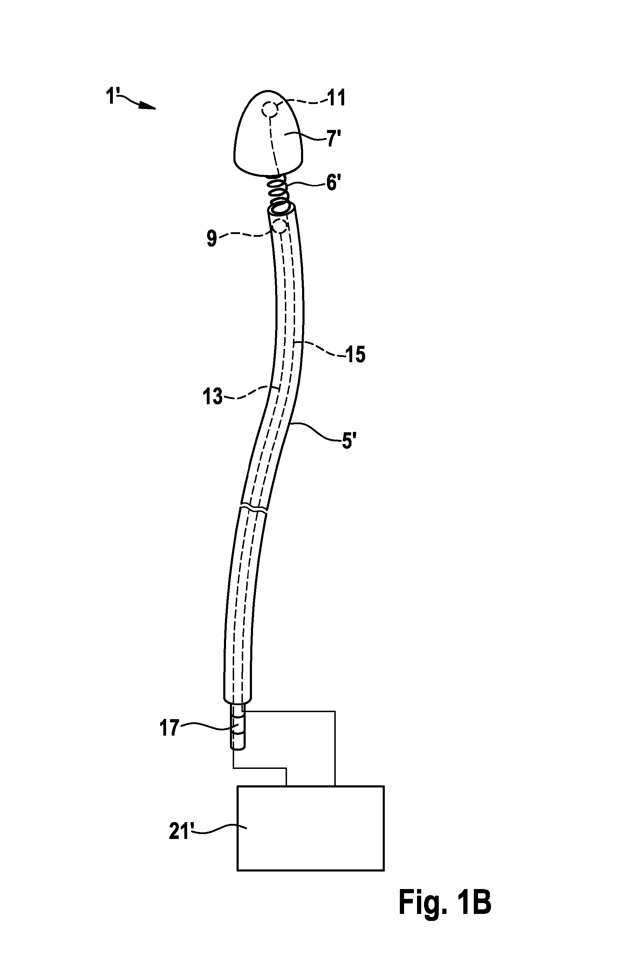

Fig. 1B zeigt als gegenüber der Ausführung nach Fig. 1A abgewandelte Ausführungsform einen Katheter 1'; hierbei sind gleiche oder funktionsgleiche Teile wie in Fig. 1A mit den gleichen Bezugsziffern wie dort bezeichnet. Der wesentlichste Unterschied besteht darin, dass hier ein axial steifer Katheterkörper 5' ohne integrierten Schutz-Abschnitt vorgesehen ist. An dessen Stelle tritt ein distal mittels einer elastischen Aufhängung 6' am Katheterkörper 5' gehaltertes Schutzelement 7'.

Im Übrigen sind Geberelement 9 und Aufnehmelement 11 hinsichtlich ihrer Positionierung gegenüber der Anordnung nach Fig. 1A vertauscht, und anstelle separater Messansteuer- und Messeinrichtungen umfasst die Anordnung eine integrierte Ansteuer- und Messeinrichtung 21'. Die Funktion der Anordnung ist im wesentlichen wie weiter oben beschrieben, und die integrierte Ansteuer- und Messeinrichtung eignet sich speziell für eine elektrische oder elektro-magnetische Ausführung der aus dem Geberelement 9 und dem Aufnehmerelement 11 bestehenden Wandlereinrichtung, in der etwa die bei einer angelegten Messspannung sich ergebende Stromstärke gemessen wird.

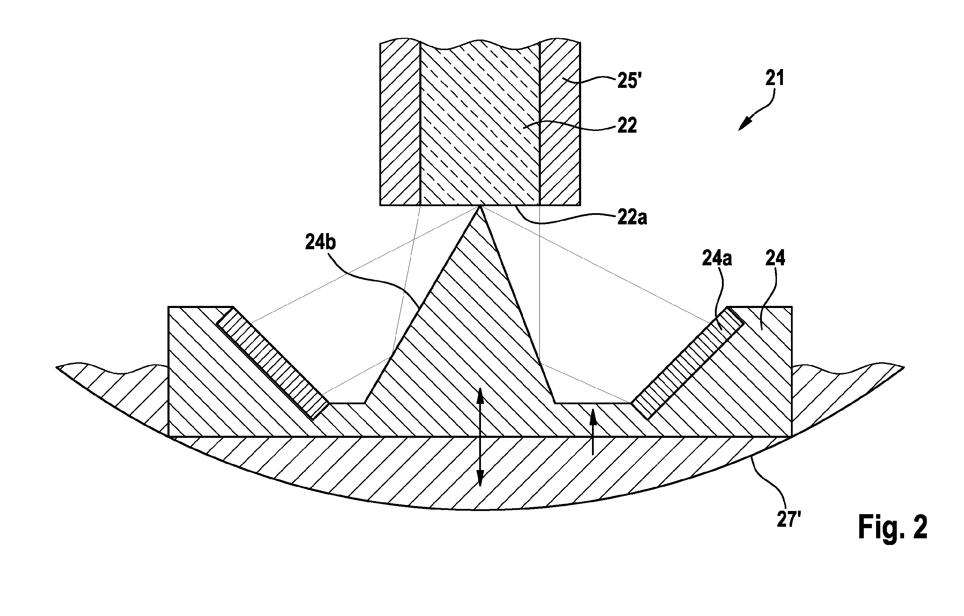

Fig. 2 zeigt als Ausschnittdarstellung des distalen Endes eines Katheters 21 eine dort implementierte optische Messtechnik. Die Bezugsziffern sind teilweise in Anlehnung an die Darstellung in Fig. 1B gewählt. In einem im Wesentlichen steifen Katheterkörper 25' ist eine Lichtleitereinrichtung 22 verlegt, deren Austrittsöffnung 22a am distalen Ende des steifen Katheterkörpers als optisches Geberelement fungiert. Am Ende des Katheterkörpers 25' ist mittels einer (nicht näher dargestellten) Aufhängung mit vorbestimmter Elastizität ein Schutzelement 27' angebracht (welches hier unmaßstäblich vergrößert und nur ausschnittsweise gezeigt ist).

In dem Schutzelement 27' befindet sich ein speziell ausgeformter Messaufnehmer-Grundkörper 24, in dem mit gleichen Winkelabständen um die verlängerte Längsachse des Katheterkörpers verteilt drei Fotodiodenarrays 24a als Aufnehmeranordnungen unter Schrägstellung zur Katheter-Längsachse eingefügt sind. Zentral im Messaufnehmer-Körper 24 befindet sich eine dreiseitige Pyramide 24b, über die das aus der Lichtaustrittsfläche 22a austretende Messlicht auf die Aufnehmeranordnungen 24a geworfen wird. Über entsprechende (nicht gezeigte) Messleitungen wird an den Fotodiodenarrays 24a jeweils ein Messsignal abgegriffen und zur Messeinrichtung der Katheteranordnung geleitet. Bei Wandkontakt der Katheterspitze verschiebt und/oder verdreht sich das Schutzelement 27', und in Folge dessen ändert sich ein Differenzsignal zwischen den Fotodiodenarrays in Abhängigkeit von Richtung und Ausmaß der Deformation, so dass diese über eine geeignete Auswertung der Messsignale bestimmt werden können.

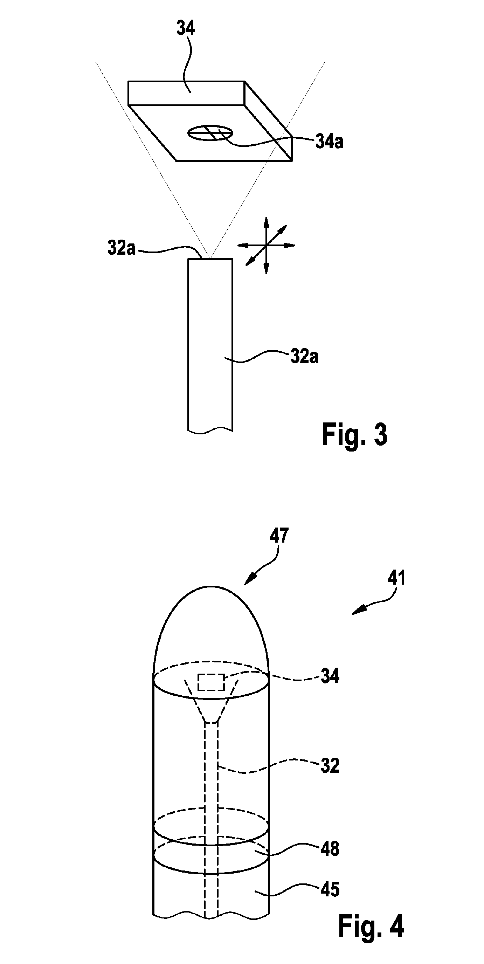

Fig. 3 zeigt skizzenartig eine weitere optoelektronische Wandlereinrichtung mit einer als Geberelement dienenden Lichtleitfaser 32 mit einer Austrittspupille 32a und einem Aufnehmerelement 34, welches eine Vier-Quadranten-Messanordnung 34a aus vier Fotodioden umfasst. Bei einer Verdrehung bzw. lateralen Verschiebung des Aufnehmerelements 24 gegenüber der Achse der Lichtleitfaser 32 und somit dem Zentrum der Lichtaustrittsposition 32a werden die einzelnen Fotodioden unterschiedlich stark ausgeleuchtet, so dass über die Auswertung eines Differenzsignals wiederum auf das Ausmaß der Verschiebung bzw. Verdrehung geschlossen werden kann. Bis zu einem gewissen Grad ermöglicht diese Anordnung eine vektorielle Auswertung.

Wie sie in den distalen Endabschnitt eines Katheters 41 eingegliedert sein kann, zeigt Fig. 4. In Anlehnung an Fig. 1A sind der Katheterkörper hier mit Ziffer 45 und dessen distaler Schutz-Abschnitt mit 47 bezeichnet. Beispielhaft ist auch eine Ringelektrode 48 gezeigt, wie sie bei einer Ausführung des Katheters 41 als Elektrodenleitung vorhanden sein kann. Es ist hier zu erkennen, dass bei einer Deformation in Achsenrichtung des Katheters und Lichtleiters 32 sich auch die empfangene Lichtintensität ändern wird, so dass über die oben erwähnten seitlichen Verschiebungen und Verdrehungen hinaus auch Stauchungen der Kathetereinrichtung in Folge eines Wandkontakts erfasst werden können.

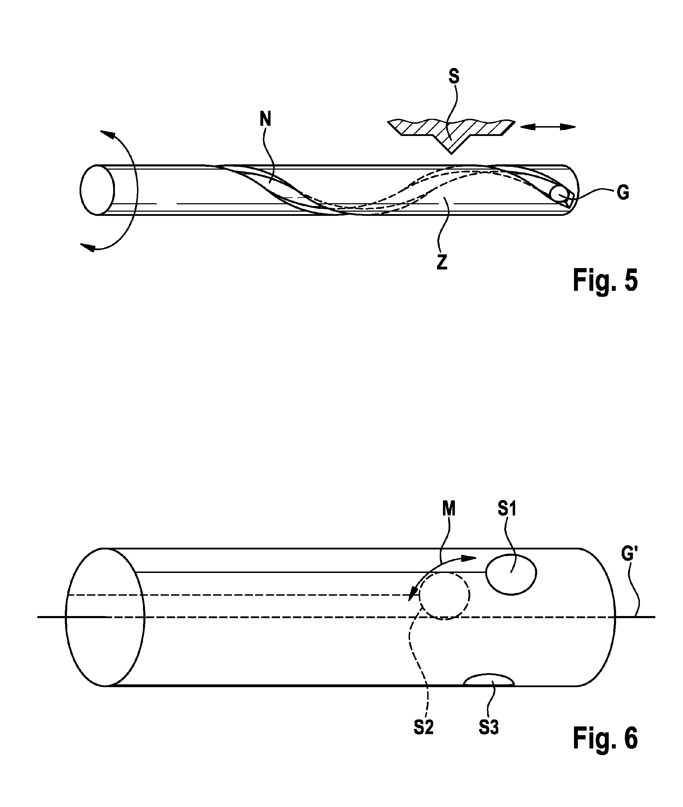

Fig. 5 ist eine Skizze zur Verdeutlichung eines weiteren Messprinzips, welches in einer Ausführung des erfindungsgemäßen Katheters eingesetzt werden kann. Hierbei ist einem ortsfesten Aufnehmerelement S ein Geberelement G zugeordnet, welches in einem Zylinder Z mit einer spiralförmig über der Wandung verlaufenden Nut N geführt ist. Wenn der Sensor mit dem beweglichen bzw. verformbaren Schutzelement oder Schutz-Abschnitt verbunden ist, so trifft bei zeitlich präzise gesteuerter Drehung der Faser ein vom Geberelement G ausgehender Lichtstrahl, je nach konkreter Position des Aufnehmerelements S, zu einem früheren oder späteren Zeitpunkt auf dieses, so dass der Zeitpunkt der Erfassung eines Signals Aufschluss über den Betrag einer Verschiebung liefert, der die Befestigungsstelle des Aufnehmerelements unterliegt. Aus dem Erfassungszeitpunkt eines Signals kann somit auf eine durch Wandkontakt verursachte Deformation der Katheterspitze geschlossen werden.

Eine weitere auf optischer Basis funktionierende Ausführungsform kann durch eine punkförmige Lichtquelle in der Mitte der Katheterspitze realisiert werden, die gegenüber einer CCD-Zeile oder einem Fotodiodenarray beweglich gelagert ist. Bei einer Krafteinwirkung von außen kommt es dann zu einer relativen Bewegung zwischen der Lichtquelle und der CCD-Zeile, wodurch aus diesem Signal auf die Krafteinwirkung rückgeschlossen werden kann. Um eine 3D-Kraft messen zu können werden hier drei CCD-Zeilen im Abstand von 120° um die Lichtquelle herum angeordnet. So kann aus der Ortsinformation der Lichtquelle zu den einzelnen CCD-Zeilen ein Kraftvektor bestimmt werden. Alternativ können z.B. auch drei Lichtquellen um eine CCD-Zeile herum angeordnet werden, wodurch sich wiederum aus den Verschiebungen der drei Lichtquellen ein 3D-Kaftvektor berechnen lässt.

Bei einer weiteren Ausführung wird die Deformation in eine spektrale Verschiebung umkodiert. Dies kann z.B. durch eine länglich angeordnete Lichtquelle geschehen, die ihr Spektrum vom distalen zum proximalen Ende ändert. Verschiebt sich diese Lichtquelle entlang eines Fensters, hinter dem sich ein Detektor befindet, kann aufgrund der detektierten Wellenlänge auf die Deformation geschlossen werden. Eine weitere Ausgestaltung funktioniert ähnlich, nur dass sich hier die Phase ändert.

Bei einer weiteren Ausführung kann ist ein Polarisationsfilter zwischen der Lichtquelle und dem Detektor untergebracht. Der Polarisationsfilter ändert seine Polarisationsrichtung entlang des Katheters, so dass sich die Polarisation des durch ein am beweglichen Katheterende angebrachten Fotodetektors empfangenen Lichtes bei einer Deformation der Katheterspitze ändert. Zur Messung der Polarisation kann das Signal über eine polarisationserhaltenden Faser zu einer Auswerteeinheit zurück übertragen werden.

Fig. 6. zeigt skizzenartig ein weiteres Messprinzip, bei dem einem zentral im Katheterkörper geführten elektrischen Geberelement G' (etwa einem stromdurchflossenen Draht) auf einem Mantelabschnitt eines verformbaren Katheterendes 3 als Spulen ausgebildete Aufnehmerelemente S1, S2, S3 (in gleichen Winkelabständen über den Umfang verteilt) gegenüberliegen. Ist der in den drei Aufnehmerspulen induzierte Strom bei einem unverformten Katheter ohne Wandkontakt gleich, so sind die erfassten Ströme bei einem verformten Katheter unterschiedlich, und ihre Auswertung ergibt Aussagen über den durch Wandkontakt hervorgerufenen Deformationszustand.

In anderen auf elektrischer Grundlage funktionierenden Ausführungen kann beispielsweise ein nahezu punktförmiges Aufnehmerelement am steifen Katheterkörper in Schleifkontakt mit einem flächigen Widerstandsbereich stehen, der fest am Schutzelement oder Schutz-Abschnitt angebracht ist und sich bei einer Deformation relativ zum punktförmigen Geberelement bewegt. Bei Anlegen einer vorbestimmten Spannung zwischen dem als Geberelement wirkenden Widerstandsbereich und dem Aufnehmerelement wird ein von der Relativposition zwischen beiden abhängiger Messstrom erfasst, und über diesen lässt sich die aktuelle Deformation des Katheterendes bestimmen. In ähnlicher Weise ist in einer weiteren Ausgestaltung auch ein kapazitiver Abgriff realisierbar.

Die Ausführung der Erfindung ist nicht auf die oben beschriebenen Beispiele und hervorgehobenen Aspekte beschränkt, sondern ebenso in einer Vielzahl von Abwandlungen möglich, die im Rahmen fachgemäßen Handelns liegen.

## Patentansprüche

1. Katheter mit einem langgestreckten und axial starren Katheterkörper, der in Bezug auf eine Gebrauchslage ein distales und ein proximales Ende hat, wobei am distalen Ende ein mit einem vorbestimmten Bewegungs- oder Verformungswiderstand versehenes bewegliches und/oder verformbares Schutzelement oder ein entsprechender Schutz-Abschnitt des Katheterkörpers angeordnet ist und ortsfest am Schutzelement oder Schutz-Abschnitt Gebermittel einer Wandlereinrichtung zur Wandlung einer Lageverschiebung in eine nicht-mechanische Größe und am Katheterkörper korrespondierende Aufnehmermittel dieser Wandlereinrichtung, oder umgekehrt, vorgesehen sind und der Katheterkörper eine Leitung zur Fortleitung eines von der Wandlereinrichtung erzeugten Messsignals zum proximalen Ende sowie einen Messanschluss am proximalen Ende aufweist.

2. Katheter nach Anspruch 1, wobei der vorbestimmte Bewegungs- oder Verformungswiderstand durch eine Federkraft einer elastischen Anbringung des Schutzelements oder eine Eigen-Elastizität des Schutzabschnitts realisiert ist.

3. Katheter nach Anspruch 1 oder 2, wobei mindestens drei Geber- oder Aufnehmerelemente um eine Längsachse oder virtuell verlängerte Längsachse des Katheterkörpers verteilt, insbesondere gleichmäßig verteilt, vorgesehen sind, um eine Lageverschiebung des Schutzelements oder Schutz-Abschnitts vektoriell zu erfassen.

4. Katheter nach Anspruch 3, wobei genau einem Geberelement genau drei Aufnehmerelemente, die insbesondere gleichmäßig mit einem Winkelabstand von je 120° um die Längsachse verteilt sind, oder ortsaufgelöst empfangende Aufnehmeranordnungen zugeordnet sind.

5. Katheter nach Anspruch 3, wobei genau drei Geberelemente, die insbesondere gleichmäßig mit einem Winkelabstand von je 120° um die Längsachse verteilt sind, einer ortsaufgelöst empfangenden Aufnehmeranordnung zugeordnet sind.

6. Katheter nach einem der vorangehenden Ansprüche, wobei die Gebermittel ein lichtemittierendes Element und die Aufnehmermittel, insbesondere auch jedes Aufnehmerelement in einer Aufnehmeranordnung, ein Fotodetektorelement aufweisen.

7. Anordnung nach Anspruch 5 und 6, wobei die Aufnehmeranordnung eine CCD-Zeile oder -Matrix aufweist.

8. Katheter nach Anspruch 6 oder 7, wobei dem oder mindestens einem lichtemittierenden Element oder dem oder mindestens einem Fotodetektor ein flächiger Farbfilter oder Polarisationsfilter mit lokal variierender Filtercharakteristik zugeordnet ist.

9. Katheter nach einem der Ansprüche 1 bis 5, wobei die Gebermittel und die Aufnehmermittel jeweils mindestens eine Elektrode oder einen elektrischen Kontakt zur Einprägung einer Messspannung und zur Erfassung eines Messstromes oder umgekehrt aufweisen.

10. Katheter nach Anspruch 9, mit mindestens einem linearen oder flächigen Elektroden- oder Widerstands-Array und einem im wesentlichen punktförmigen Abtast-Kontakt oder kapazitiven Abgriff als Geber- und Aufnehmermittel, wobei eines von Elektroden- bzw. Widerstands-Array oder Abtast-Kontakt ortsfest am starren Katheterkörper und das andere ortsfest am Schutzelement oder Schutz-Abschnitt angebracht ist.

11. Katheter nach einem der vorangehenden Ansprüche, wobei den Geber- oder Aufnehmermitteln Zeitsteuermittel zu einer vorbestimmten zeitlichen Steuerung ihrer Aktivierung oder ihres Ortes auf einer vorbestimmten Bewegungsbahn im Bereich des Schutzelements oder Schutz-Abschnitts zugeordnet und die Aufnehmermittel zur zeitaufgelösten Signalaufnahme ausgebildet sind.

12. Katheter nach einem der vorangehenden Ansprüche, ausgebildet als Elektrodenleitung mit mindestens einer Stimulations- und/oder Abfühlelektrode.

13. Katheteranordnung mit einem Katheter nach einem der vorangehenden Ansprüche und einer an den Messanschluss angeschlossenen Messeinrichtung zur Bestimmung einer Lageverschiebung des Schutzelements oder Schutz-Abschnittes und/oder einer hierauf einwirkenden Andruckkraft.

14. Katheteranordnung nach Anspruch 13, mit einem Katheter nach Anspruch 11 oder 12, wobei die Zeitsteuermittel zu einer vorbestimmten zeitlichen Steuerung der Aktivierung oder des Ortes der Geber- oder Aufnehmermittel als Komponente der an den Katheter angeschlossen Messeinrichtung ausgeführt sind.
